(19) **Europäisches Patentamt**
**European Patent Office**
**Office européen des brevets**

(11) **EP 4 671 705 A1**

(12) **EUROPÄISCHE PATENTANMELDUNG**

(43) Veröffentlichungstag:
**31.12.2025 Patentblatt 2026/01**

(21) Anmeldenummer: **24184279.8**

(22) Anmeldetag: **25.06.2024**

(51) Internationale Patentklassifikation (IPC):
***G01F 25/00*** *(2022.01)* ***A61M 15/08*** *(2006.01)*
***B05B 11/00*** *(2023.01)*

(52) Gemeinsame Patentklassifikation (CPC):
**G01F 25/0092; A61M 11/006; A61M 15/08;**
**A61M 2209/02; B05B 11/1001**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Harro Höfliger**
**Verpackungsmaschinen GmbH**
**71573 Allmersbach im Tal (DE)**

(72) Erfinder:
• **Pohl, Bernd**
**71573 Allmersbach im Tal (DE)**
• **Pröger, Bernd**
**71573 Allmersbach im Tal (DE)**

(74) Vertreter: **Schmid, Barbara**
**Bismarckstraße 36**
**74074 Heilbronn (DE)**

(54) **VORRICHTUNG UND VERFAHREN ZUM PRÜFEN DES SPRÜHSTRAHLS EINER BAUGRUPPE FÜR SPRÜHAPPLIKATOREN**

(57) Die Erfindung betrifft ein Verfahren zum Prüfen des Sprühstrahls (10, 12) einer Baugruppe für Sprühapplikatoren mit folgenden Verfahrensschritten:
- die zu prüfende Baugruppe wird in einer Aufnahmeeinheit einer Prüfvorrichtung positioniert und mit einem Flüssigkeitsreservoir verbunden,
- die zu prüfende Baugruppe wird gespannt und saugt dabei ein bestimmtes Volumen des Prüf-Fluids aus dem Flüssigkeitsreservoir an,
- die zu prüfende Baugruppe wird betätigt, so dass ein Sprühstrahl (10, 12) entsteht,

- der Sprühstrahl (10, 12) verdunkelt ein Lichtband oder einen Lichtstrahl einer Lichtschranke,
- die Verdunklung des Lichtbands beziehungsweise des Lichtstrahls wird mittels einer Sensoreinheit gemessen und die Messwerte (14) in Abhängigkeit von der Zeit registriert und ausgewertet,
- aufgrund der ermittelten Messwerte (14) wird der Beginn ($t3$) des Sprühstrahls (10) und/oder das Ende ($t4$) des Sprühstrahls (12) und/oder die Dauer (24) des Sprühstrahls (10, 12) berechnet.

FIG.1

EP 4 671 705 A1

**Beschreibung**

TECHNISCHES GEBIET

Die Erfindung betrifft eine Vorrichtung und ein Verfahren zum Prüfen des Sprühstrahls einer Baugruppe von Sprühapplikatoren. Bei derartigen Sprühapplikatoren kann es sich insbesondere um Hochdruckzerstäuber wie Inhalatoren handeln, aber auch um Sprays wie beispielsweise um Nasen- oder Augensprays. Sprühapplikatoren werden vor allem dann verwendet, wenn die Verabreichung eines Wirkstoffs dosisabhängig und/oder abhängig von der Tröpfchengröße ist.

Inhalatoren sind medizinische Geräte (Applikatoren) zur Erzeugung von Aerosolen oder Dämpfen; die Aerosole oder Dämpfe können dann von Patienten eingeatmet werden. Inhalatoren werden insbesondere bei der Behandlung von verschiedenen Atemwegserkrankungen wie beispielsweise von Asthma oder von COPD (Chronic Obstructive Pulmonary Disease, chronisch-obstruktive Lungenkrankheit) eingesetzt. Durch die Inhalatoren können Wirkstoffe in die Lunge gelangen, die dort lokal begrenzt wirken. Dadurch können Nebenwirkungen vermindert werden; auch ist häufig lediglich eine geringere Dosis des Wirkstoffs erforderlich. Der Wirkstoff kann über die Alveolen der Lunge auch in den Blutkreislauf gelangen und dort systemisch wirken. Auch hier sind häufig geringere Dosen ausreichend, da der First-Pass-Effekt der Leber umgangen wird. Darüber hinaus gelangen die Wirkstoffe aufgrund der großen Resorptionsfläche der Lunge schneller in den Blutkreislauf, so dass der Effekt sehr rasch eintritt.

STAND DER TECHNIK

Die Inhalatoren können beispielsweise als Soft Mist Inhaler (SMI, Hochdruckzerstäuber) ausgebildet sein. Derartige Inhalatoren werden insbesondere in der WO 91/14468 A1, der WO 97/12687 A1 oder der WO 2009/047173 A2 beschrieben. Die Soft Mist Inhaler weisen einen Zerstäuber mit einer mechanischen Pumpe auf, durch die eine langanhaltende feine Sprühwolke eines Fluids erzeugt werden kann. Bei dem Fluid handelt es sich in der Regel um eine wässrige oder ethanolische Lösung des Wirkstoffs. Dabei werden keine Treibmittel für die Erzeugung des Sprühnebels benötigt. Das Fluid liegt in der Regel in einer Kartusche vor. Nach dem Verbrauch einer solchen Kartusche kann diese häufig zumindest einige Male ersetzt werden.

Der Inhalator selbst besitzt eine stabile Kapillare, die in die Kartusche mit dem Fluid eingeführt wird. Die Kapillare taucht ein Stück weit in das Fluid ein, so dass sich dieses durch Kapillarkräfte innerhalb der Kapillare nach oben zieht. Im oberen Bereich der Kapillare sitzt ein Hochdruck-Rückschlagventil zur Dosierung des Fluids. Dieses Rückschlagventil dient dabei sowohl zur Absperrung als auch zur Steuerung des Durchflusses des Fluids innerhalb der Kapillare. Das Fluid kann innerhalb der Kapillare nach oben fließen, jedoch nicht wieder zurück nach unten und damit wieder zurück in die Kartusche.

Die Kapillare endet mit ihrem oberen Ende in einer Hochdruck-Pumpenbaugruppe, die die zentrale Einheit der Soft Mist Inhaler - und auch der anderen Sprühapplikatoren - darstellt. Die korrekte Montage dieser Pumpenbaugruppe ist für eine einwandfreie Funktion des Inhalators maßgeblich, so dass bereits geringfügige Fehler oder Abweichungen zu einer Fehlfunktion des Inhalators führen können.

Nach der vollständigen Montage des Inhalators findet in der Regel eine Qualitätsprüfung statt. Durch die Qualitätsprüfung können Fehlfunktionen des Inhalators noch vor der Auslieferung entdeckt werden, so dass keine nicht-spezifikationsgerechten Inhalatoren in den Vertrieb gelangen. Darüber hinaus muss auch sichergestellt werden, dass die Dosierung des Fluids reproduzierbar erfolgt. Eine solche Überprüfung der Inhalatoren kann im Rahmen einer Vollprüfung (100%-Prüfung) erfolgen, bei der sämtliche Inhalatoren routinemäßig entsprechend getestet werden. Es wäre auch möglich, die Überprüfung der Inhalatoren im Rahmen von Stichprobenprüfungen vorzunehmen.

Aus der WO 2017/060328 A1 beziehungsweise der US 2019/223418 A1 ist ein Prüfsystem und ein Prüfverfahren bekannt, bei der der fertig montierte Inhalator mit einem Prüf-Fluid getestet wird. Der fertige Inhalator wird dazu an eine Prüf-Kartusche mit dem Prüf-Fluid angeschlossen und mehrere Male maschinell betätigt. Dabei werden verschiedene Messungen, insbesondere hinsichtlich der Verteilung des Sprühnebels und der Menge des zerstäubten Fluids vorgenommen. Sofern der Inhalator bei dieser Prüfung keine korrekten Messwerte liefert, wird der Inhalator aussortiert.

Um Fehlfunktionen einzelner wichtiger Bauteile möglichst frühzeitig erkennen und beheben zu können, sind darüber hinaus auch Einzelprüfungen bestimmter Bauteile bekannt. Aus der DE 2020 210 028 U1 ist eine Vorrichtung zum Prüfen von an Kapillaren befestigten Hochdruckrückschlagventilen bekannt. Bei einer Undichtigkeit des Hochdruckrückschlagventils kann dieses unmittelbar aussortiert werden. Aus der EP 4 109 066 A1 ist eine Vorrichtung zum Prüfen der Dichtigkeit einer Pumpenbaugruppe für Hochdrucksprühapplikatoren bekannt. Die Pumpenbaugruppe kann dadurch separat von den weiteren Bauteilen der Sprühapplikatoren auf Dichtigkeit überprüft werden, so dass lediglich spezifikationsgerechte Pumpenbaugruppe für die Endmontage verwendet werden.

DARSTELLUNG DER ERFINDUNG

Ausgehend von diesem vorbekannten Stand der Technik liegt der Erfindung die Aufgabe zugrunde, ein verbessertes Prüfverfahren und eine verbesserte Prüfvorrichtung zur Überprüfung des Sprühstrahls einer Baugruppe für Sprühapplikatoren anzugeben.

Das

Das erfindungsgemäße Prüfverfahren ist durch die Merkmale des Hauptanspruchs 1 gegeben. Die erfindungsgemäße Prüfvorrichtung ist durch die Merkmale des nebengeordneten Anspruchs 14 gegeben. Sinnvolle Weiterbildungen der Erfindung sind Gegenstand von sich an diese Ansprüche anschließenden weiteren Ansprüchen.

Das erfindungsgemäße Verfahren zum Prüfen des Sprühstrahls einer Baugruppe für Sprühapplikatoren weist folgende Verfahrensschritte auf:

1. Die zu prüfende Baugruppe wird in einer Aufnahmeeinheit einer Prüfvorrichtung positioniert und mit einem Flüssigkeitsreservoir verbunden.
2. Die zu prüfende Baugruppe wird gespannt und saugt dabei ein bestimmtes Volumen des Prüf-Fluids aus dem Flüssigkeitsreservoir an.
3. Die zu prüfende Baugruppe wird betätigt, so dass ein Sprühstrahl entsteht.
4. Der Sprühstrahl verdunkelt ein Lichtband oder einen Lichtstrahl einer Lichtschranke.
5. Die Verdunklung des Lichtbands wird mittels einer Sensoreinheit gemessen und die Messwerte werden in Abhängigkeit von der Zeit registriert und ausgewertet.
6. Aufgrund der ermittelten Messwerte wird der Beginn des Sprühstrahls (t3) und/der das Ende des Sprühstrahls (t4) und/oder die Dauer des Sprühstrahls berechnet.

Bei dem Lichtband oder dem Lichtstrahl kann es sich vorzugsweise um einen Laserstrahl oder um einen gefächerten Laserstrahl handeln.

Die Auswertung der Messwerte kann insbesondere per Software und/oder Hardware erfolgen.

Nach dem Auslösen des Sprühstrahls dauert es in der Regel wenige Hundert Millisekunden, bis sich ein Druck aufgebaut hat. Dann erfolgt ein schlagartiger Anstieg des Verdunklungswerts, der mit dem Beginn des Sprühstrahls korreliert. Bei einem stabilen Sprühstrahl sollte der Verdunklungswert für eine gewisse Zeit etwa konstant bleiben. Anschließend fällt der Verdunklungswert zügig wieder ab.

Aus den aufgezeichneten Messwerten für den Verdunklungswert können über mehrere Algorithmen die Punkte P3 (Zeitpunkt t3) als der Beginn des Sprühstrahls und P4 (Zeitpunkt t4) als das Ende des Sprühstrahls (Unterschreiten einer definierten Schwelle des Verdunklungswerts) ermittelt werden. Dadurch kann zunächst die Dauer des Sprühstrahls berechnet werden. Aus diesen Werten können wichtige Erkenntnisse über die Qualität der zu prüfenden Baugruppe gewonnen werden. Insbesondere kann kontrolliert werden, ob die entsprechenden Messwerte jeweils in einem vorgegebenen Referenzbereich liegen oder nicht. Sofern die Messwerte innerhalb des vorgegebenen Referenzbereichs liegen, hat die zu prüfende Baugruppe den ersten Teil des Prüfungsverfahrens bestanden. Sofern die Messwerte dagegen außerhalb des vorgegebenen Referenzbereichs liegen, erfüllt die Baugruppe nicht die erforderlichen Spezifikationen und wird ausgeschieden.

Die Verdunklungswerte können dazu insbesondere in Form eines Diagramms, insbesondere in Form eines Liniendiagramms ausgegeben werden. Aus dem Linienverlauf können dadurch neben den bereits erwähnten Werten weitere Erkenntnisse gewonnen werden, durch die dann Rückschlüsse auf die Güte der geprüften Baugruppe geschlossen werden können. Alternativ oder zusätzlich dazu kann auch eine tabellarische Ausgabe der Verdunklungswerte erfolgen.

Insbesondere kann in diesem Zusammenhang kontrolliert werden, ob der abfallende Linienverlauf der Verdunklungswerde zum Ende des Sprühstrahls hin in einem vorgegebenen Referenzbereich liegen oder nicht. Fallen die Werte zu schnell ab, ist die Düse der geprüften Baugruppe zu weit. Dies kann beispielsweise dann der Fall sein, wenn der Düsenquerschnitt außerhalb der Toleranz liegt. Fallen die Werte dagegen zu langsam ab, ist die Düse der geprüften Baugruppe zu eng. Dies kann beispielsweise bei einer Verschmutzung der Düse oder durch einen Produktionsfehler der Fall sein.

In einer besonders bevorzugten Ausführungsform können die ermittelten Messwerte zumindest im Bereich zwischen dem Beginn des Sprühstrahls (P3, t3) und dem Ende des Sprühstrahls (P4, t4) integriert werden, anschließend kann das Integral der Messwerte ausgewertet werden.

Durch eine Integration der Messwerte während der Dauer des Sprühstrahls kann anschließend die Qualität des Sprühstrahls berechnet werden. Das Integral der Messwerte kann vorzugsweise in Form eines Liniendiagramms ausgegeben werden. Aus dem Linienverlauf des Integrals können somit weitere Daten berechnet werden; auch der Linienverlauf des Integrals kann Rückschlüsse über die geprüfte Baugruppe ermöglichen. Alternativ oder zusätzlich dazu kann auch eine tabellarische Ausgabe des Integrals erfolgen.

In diesem Zusammenhang kann insbesondere der Anstieg des Integrals und der diesbezügliche Linienverlauf während des Anstiegs des Integrals betrachtet werden. Beispielsweise kann kontrolliert werden, ob der Anstieg des Integrals in einem vorgegebenen Referenzbereich liegt oder nicht. Alternativ oder zusätzlich dazu kann kontrolliert werden, ob der Anstieg des Integrals etwa linear verläuft. Ein annähernd linearer Anstieg des Integrals setzt voraus, dass ein konstanter Sprühnebel erzeugt wurde. Dies ist nur dann der Fall, wenn der Kolben der geprüften Baugruppe stetig läuft.

Alternativ oder zusätzlich dazu kann kontrolliert werden, ob das Maximum des Integrals in einem vorgegebenen Referenzbereich liegt oder nicht.

In einer besonders vorteilhaften Ausführungsform kann ein weiterer Endpunkt des Sprühstrahls aus dem Integral bestimmt werden. Dazu kann beispielsweise der Übergangspunkt P7 des Integrals (Zeitpunkt t7) bestimmt werden. Bei diesem Übergangspunkt P7 des Integrals kann es sich um den Bereich des Übergangs zwischen einem annähernd linearen Anstieg des Integrals in einen gekrümmten Anstieg des Integrals handeln. Alternativ oder zusätzlich dazu kann das Überschreiten einer definierten Prozentzahl des Maximus des Integrals als Ende P6 des Sprühstrahls (Zeitpunkt t6) bestimmt werden. Anschließend kann kontrolliert werden, ob die Punkte P6 und/oder P7 in einem vorgegebenen Referenzbereich liegen oder nicht. Darüber hinaus kann der zeitliche Abstand zwischen dem Beginn des Sprühstrahls (t3) und den Punkten P7 beziehungsweise P6 berechnet werden. Auch hier kann wieder kontrolliert werden, ob dieser zeitliche Abstand in einem vorgegebenen Referenzbereich liegt oder nicht.

Auf der Basis der oben beschriebenen Auswertungen kann jeweils eine manuelle oder automatische Bewertung der Baugruppe erfolgen.

Das erfindungsgemäße Prüfverfahren kann in den Montageprozess des Sprühapplikators eingebunden werden, so dass jeder Sprühapplikator durch das Prüfverfahren kontrolliert werden kann (100%-Prüfung). Alternativ dazu wäre auch eine Stichproben-Prüfung der Sprühapplikatoren möglich.

Bei einer Integration in den Montageprozess ist es in der Regel sinnvoll, jede Baugruppe nur wenige Male zu überprüfen. Das Prüfverfahren wird in diesem Fall für jede Baugruppe vorzugsweise drei Mal durchgeführt, so dass eine gewisse statistische Sicherheit besteht. Auf diese Weise kann insbesondere auch eine Reproduzierbarkeit der Messwerte überprüft werden, so dass eine optimale Qualitätsprüfung der entsprechenden Baugruppe realisiert werden kann. Es wäre jedoch auch möglich, jede Baugruppe lediglich einmalig zu überprüfen.

Alternativ dazu könnte das erfindungsgemäße Prüfverfahren auch deutlich häufiger mit derselben Baugruppe durchgeführt werden. Eine solche mehrmalige Durchführung des Prüfverfahrens kann insbesondere während der Erprobungsphase des Montageprozesses sinnvoll sein. Darüber hinaus kann auf diese Weise die Qualität des Primens (Entlüften der Pumpe beim ersten Befüllen der zunächst trockenen Pumpenkammer) erfasst und bewertet werden. Dabei können mehrere Messungen hintereinander durchgeführt werden, wobei für jede Messung die Dauer des Sprühstrahls berechnet werden kann. Die jeweils ermittelten Dauern des Sprühstrahls können anschließend in der Reihenfolge der Messungen ausgewertet werden. So kann beispielsweise ermittelt werden, mit wie vielen Auslösungen die Baugruppe die volle Sprühzeit erreicht.

Die erfindungsgemäße Vorrichtung zum Prüfen des Sprühstrahls einer Baugruppe für Sprühapplikatoren besitzt zumindest eine Aufnahmeeinheit für die zu prüfende Baugruppe, zumindest ein Flüssigkeitsreservoir für ein Prüf-Fluid, sowie eine Betätigungseinheit für die zu prüfende Baugruppe, so dass die Baugruppe mit dem Prüf-Fluid befüllt und ein Sprühstrahl erzeugt werden kann. Darüber hinaus ist eine Lichtschranke mit einem Lichtband oder einem Lichtstrahl vorhanden, die so vor der zu prüfenden Baugruppe positioniert ist, dass der von der zu prüfenden Baugruppe erzeugte Sprühstrahl auf das Lichtband oder den Lichtstrahl trifft. Die Verdunklung des Lichtbandes beziehungsweise des Lichtstrahls kann mittels einer Sensoreinheit erfasst werden. Erfindungsgemäß ist eine Integriereinheit zum Integrieren der von der Sensoreinheit aufgezeichneten Messwerte vorhanden. Darüber hinaus ist zumindest eine Auswerteeinheit vorhanden, mittels der die aufgezeichneten Messwerte und das Integrals der aufgezeichneten Messwerte ausgewertet werden kann.

Bei dem Lichtband oder dem Lichtstrahl kann es sich vorzugsweise um einen Laserstrahl oder um einen gefächerten Laserstrahl handeln.

Bei der Integriereinheit und/oder der Auswerteeinheit kann es sich insbesondere um eine entsprechende Software handeln, die beispielsweise in einer Steuereinheit integriert sein kann.

Dabei kann es ausreichend sein, lediglich eine einzelne Auswerteeinheit vorzusehen, mittels der sowohl die aufgezeichneten Messwerte als auch das Integrals der aufgezeichneten Messwerte ausgewertet werden kann. Alternativ dazu kann auch eine erste Auswerteeinheit vorhanden sein, mittels der zunächst lediglich die aufgezeichneten Messwerte ausgewertet werden. Das Integral der aufgezeichneten Messwerte kann anschließend mittels einer zweiten Auswerteeinheit ausgewertet werden.

Vorzugsweise kann eine Anzeigeeinheit zum Ausgeben der aufgezeichneten Messwerte und/oder zum Ausgeben des Integrals der aufgezeichneten Messwerte vorhanden sein. Die Ausgabe der jeweiligen Daten kann dabei insbesondere in Form von Liniendiagrammen und/oder in Tabellenform erfolgen.

Darüber hinaus können die aufgezeichneten Messwerte und/oder das Integral der aufgezeichneten Messwerte und/oder die Auswertung der Messwerte durch die Auswerteeinheit gegebenenfalls inklusive einer Gut/Schlecht-Bewertung an eine Steuereinheit übermittelt werden.

Vorzugsweise kann die Sensoreinheit zur Messung des Verdunklungswerts der Lichtschranke mit dem Betätigen der zu prüfenden Baugruppe gestartet werden.

Bei dem Flüssigkeitsreservoir kann es sich beispielsweise um eine mit einem Prüf-Fluid gefüllte Kartusche handeln, die in die zu prüfende Baugruppe eingesetzt werden kann. Alternativ dazu wäre auch ein mit dem Prüf-Fluid befüllter Prüfkörper denkbar, der ebenfalls in dar zu prüfende Baugruppe einsetzbar ist. Grundsätzlich wäre auch ein größeres Flüssigkeitsreservoir möglich, das für mehrere zu prüfende Baugruppen gleichzeitig genutzt werden kann. In diesem Fall könnten die einzelnen zu prüfenden Baugruppen jeweils über ein Kopplungselement an dem Flüssigkeitsreservoir angeschlossen werden. Bei diesem Kopplungselement kann es sich beispielsweise um die Kapillare mit dem integrieren Rückschlagventil handeln, die ein wichtiger Bestandteil der Sprühapplikatoren darstellt. Alternativ dazu kann es sich bei dem Kopplungselement auch um einen Schlauch handeln, der an die Kapillare oder den Hohlkolben der Baugruppe angeschlossen werden kann.

Die Betätigungseinheit kann vorzugsweise einen elektrischen oder pneumatischen Auslöser aufweisen.

Bei der zu prüfenden Baugruppe kann es sich insbesondere um einen funktional fertigen Sprühapplikator handeln. Dadurch kann eine Endkontrolle des Sprühapplikators realisiert werden. In diesem Fall dürfte der fertig montierte Sprühapplikator noch keinen Mundschutz-Deckel aufweisen, da dieser eine Prüfung des Sprühstrahls verhindern würde. Auch könnte in den fertig montierten Sprühapplikator bereits eine Kartusche mit dem Wirkstoff eingesetzt sein.

Der ausgestoßene Sprühnebel sollte jeweils abgesaugt werden, um Kontaminationen und Gefährdungen zu vermeiden und einen Explosions-Schutz zu gewährleisten. Die Absaugung könnte so gestaltet sein, dass dadurch gleichzeitig eine Ausrichtung des Sprühnebels erreicht werden kann. Dadurch kann die Reproduzierbarkeit der Messungen erhöht werden.

Die Bedienung der Prüfvorrichtung kann manuell, semiautomatisch oder vollautomatisch umgesetzt werden. Die Prüfvorrichtung kann in den Montageprozess des Inhalators so integriert werden, dass jede Baugruppe von der Prüfvorrichtung getestet wird, so dass eine 100%-Prüfung erfolgt. Es wäre jedoch auch eine Stichprobenprüfung möglich.

Weitere Vorteile und Merkmale der Erfindung sind den in den Ansprüchen ferner angegebenen Merkmalen sowie den nachstehenden Ausführungsbeispielen zu entnehmen.

KURZE BESCHREIBUNG DER ZEICHNUNG

Die Erfindung wird im Folgenden anhand der in der Zeichnung dargestellten Ausführungsbeispiele näher beschrieben und erläutert. Es zeigen:

Fig. 1 ein schematisches Liniendiagramm der gemessenen Verdunklungswerte mit eingezeichneten Referenzbereichen,

Fig. 2 ein schematisches Liniendiagramm gemäß Fig. 1, bei dem der Abfall der Verdunklungswerte zum Ausschluss der geprüften Baugruppe führen würde,

Fig. 3 ein weiteres schematisches Liniendiagramm gemäß Fig. 1, bei dem der Abfall der Verdunklungswerte zum Ausschluss der geprüften Baugruppe führen würde,

Fig. 4 ein schematisches Liniendiagramm gemäß Fig. 1 mit zusätzlich eingezeichnetem Integral der Messwerte,

Fig. 5 ein schematisches Liniendiagramm gemäß Fig. 4, bei dem der Anstieg des Integrals zum Ausschluss der geprüften Baugruppe führen würde,

Fig. 6 ein weiteres schematisches Liniendiagramm gemäß Fig. 4, bei dem der Anstieg des Integrals zum Ausschluss der geprüften Baugruppe führen würde,

Fig. 7 ein schematische Liniendiagramm gemäß Fig. 4 mit Bestimmung des Wendepunkts des Integrals,

Fig. 8 ein schematisches Diagramm der Sprühzeit $t_s$ abhängig von der Anzahl der Primehübe,

Fig. 9 ein schematische Diagramm gemäß Fig. 8, das zum Ausschluss der geprüften Baugruppe führen würde, und

Fig. 10 eine schematische Darstellung der erfindungsgemäßen Vorrichtung zum Prüfen des Sprühstrahls einer Baugruppe für Sprühapplikatoren.

WEGE ZUM AUSFÜHREN DER ERFINDUNG

Bei dem erfindungsgemäßen Prüfverfahren wird zunächst die zu prüfende Baugruppe mit einer definierten Dosis des Prüf-Fluids befüllt. Dazu kann die zu prüfende Baugruppe an ein entsprechendes Flüssigkeitsreservoir angeschlossen werden. Beim Aufziehen der zu prüfenden Baugruppe wird die interne Feder der Baugruppe gespannt und das Prüf-Fluid aus dem Reservoir angesaugt. Durch einen elektrischen oder pneumatischen Auslöser wird anschließend der Auslösetaster an der zu prüfenden Baugruppe gedrückt. Gleichzeitig beginnt die Aufzeichnung der Verdunklungswerte des Lichtbandes oder des Lichtstrahls durch die Sensoreinheit.

Durch das Auslösen der zu prüfenden Baugruppe wird der federvorgespannte Kolben freigegeben und fördert unter einem hohen Druck das angesaugte Volumen des Prüf-Fluids durch eine Mikrodüse der Baugruppe. Diese Mikrodüse zerstäubt die Flüssigkeit zu einem feinen Sprühstrahl. Der Sprühstrahl trifft auf das Lichtband, so dass dieses verdunkelt wird. Für eine einstellbare Zeit werden die Verdunklungswerte von der Sensoreinheit gemessen und abgespeichert. Die Speicherung der Verdunklungswerte kann dabei durch die Sensoreinheit oder durch eine separate Steuerungseinheit erfolgen.

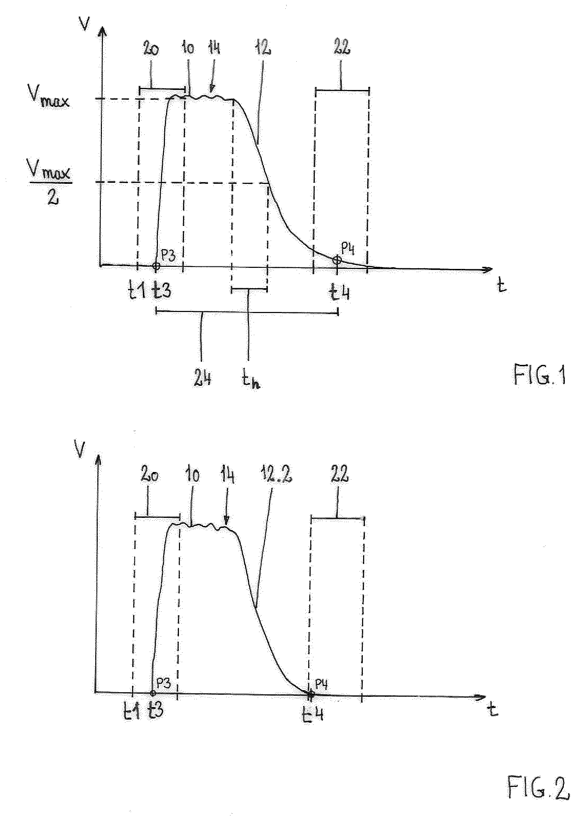

Die gemessenen Verdunklungswerte V können anschließend in einem Liniendiagramm in Relation zur verstrichenen Zeit ausgegeben werden. Dabei weisen die Verdunklungswerte V bei einer spezifikationsgerechten Baugruppe einen typischen Verlauf auf, der in Fig. 1 schematisch dargestellt ist.

Nach dem Auslösen der Baugruppe (Zeitpunkt t1) baut sich in der Regel in kurzer Zeit der Druck in der Pumpe und damit der Sprühstrahl auf. Dadurch entsteht ein steiler Anstieg der Verdunklungswerte auf einen nahezu stabilen Sprühstrahl 10 mit maximalen Verdunklungswerten ($V_{max}$). Ist der Kolbenweg zu Ende, entspannt sich das System und der Sprühstrahl und damit auch die Verdunklungswerte nehmen ab (abfallender Sprühstrahl 12).

Über mehrere Algorithmen wird aus den aufgezeichneten Messwerten 14 zunächst der Beginn des Sprühstrahls (Punkt P3, Zeitpunkt t3) und das Ende des Sprühstrahls (Punkt P4, Zeitpunkt t4) gesucht. Dazu werden die Verdunklungswerte zunächst skaliert, indem Minimum und Maximum der Verdunklungswerte gesucht werden. Die Messwerte zwischen diesen beiden Extremen werden entsprechend skaliert. Der absolut gemessene Verdunklungswert kann nicht herangezogen werden, da dieser nicht normiert ist. Darüber hinaus variiert der absolut gemessene Verdunklungswert abhängig von dem exakten Aufbau für das Prüfungsverfahren und der genauen Positionierung der Lichtschranken. Durch die Skalierung können auch Streuungen in den Messungen eliminiert werden. Wird die Messung in einer Dunkelkammer durchgeführt, können externe Lichteinflüsse zusätzlich eliminiert werden.

Der Zeitpunkt t3 entspricht dabei dem Beginn des Sprühstrahls nach dem Auslösen der zu prüfenden Baugruppe. Der Zeitpunkt t3 ist dabei erkennbar durch den steilen Anstieg des Verdunklungswerts. Der Zeitpunkt t4 markiert das Ende des Sprühstrahls. Der Zeitpunkt t4 wird in der Regel durch das Unterschreiten einer gewissen Schwelle des Verdunklungswerts bestimmt.

Um eine Baugruppe als spezifikationsgerecht zu bewerten, muss der Zeitpunkt t3 in einem vorgegebenen Referenzbereich 20 liegen. Der Referenzbereich 20 wird dabei durch den Auslösezeitpunkt t1 definiert. Der Zeitpunkt t4 muss ebenfalls in einem vorgegebenen Referenzbereich 22 liegen. Darüber hinaus kann der Zeitabstand 24 zwischen t3 und t4 berechnet werden. Auch dieser Wert 24 (Sprühzeit $t_s$) muss in einem vorgegebenen Referenzbereich liegen, wenn es sich um eine spezifikationsgerechte Baugruppe handelt.

Die Sprühzeit $t_s$ ist in erster Näherung proportional zum angesaugten beziehungsweise abgemessenen Volumen MV (Metered Volume) der Pumpe bezogen auf den als konstant angenommenen Volumenstrom $V_D$ der Düse.

$$t_s = \frac{MV}{\dot{V}_D}$$

Das abgemessene Volumen MV ist das vom Kolben beim Auslösen der Baugruppe verdrängte Volumen und kann aus dem Kolbenquerschnitt AK und dem Kolbenhub h berechnet werden.

$$MV = A_K \times h$$

Der Volumenstrom $V_D$ durch die Düse ergibt sich aus der Formel:

$$\dot{V}_D = A_D \times v_D$$

Dadurch ergibt sich die Sprühzeit $t_s$ aus der Formel:

$$t_s = \frac{A_K}{A_D} \times \frac{h}{v_D}$$

Die Austrittsgeschwindigkeit $v_D$ des Mediums aus der Düse berechnet sich bei einer Dichte $\rho$ des Mediums für stationäre Strömungen ohne Betrachtung der Verluste durch folgende Formel:

$$v_D = \sqrt{2\frac{p}{\rho}}$$

Der Druck p berechnet sich aus der mittleren Kolbenkraft $F_K$ und dem Kolbenquerschnitt $A_K$ durch folgende Formel:

$$p = \frac{F_K}{A_K}$$

Die Sprühzeit ts ergibt sich somit durch die folgende Formel:

$$t_s = \frac{A_K}{A_D} \times \frac{h}{\sqrt{2 \times \frac{F_K}{A_K \times \rho}}}$$

Somit können über die Messung der Sprühzeit $t_s$ größere Abweichungen unmittelbar erkannt und die geprüften Baugruppen als schlecht ausgegeben werden.

Aufgrund der Multiparameterabhängigkeit kann die Ursache der Abweichung in der Regel zunächst nicht eindeutig zugeordnet werden. Dies kann jedoch gegebenenfalls durch eine Nachinspektion der einzelnen Komponenten der Baugruppe erfolgen.

Neben den geometrischen Größen Kolbenquerschnitt $A_K$, Düsenquerschnitt $A_D$ und Kolbenhub h geht auch die Kolbenkraft $F_K$ in die Sprühzeit $t_s$ ein. In der Praxis stellen sich noch Reibungskräfte ein, die sich dann in einer Reduktion der wirksamen Kolbenkraft $F_K$

und somit in der Sprühzeit $t_s$ äußern.

Neben den geometrischen und kräftemäßigen Abhängigkeiten der Sprühzeit $t_s$ können Abweichungen vom Soll auch Undichtigkeiten im Bereich des Rückschlagventils oder der Elastomer-Dichtungen erkannt werden. Dies liegt daran, dass das theoretisch mögliche "Metered Volume" MV ($MV = A_K \times h$) bei auftretenden Leckage-Verlusten um das "Leckage Volume" LV reduziert wird:

$$MV = A_K \times h - LV$$

Aufgrund der Proportionalität zwischen der Sprühzeit und dem "Metered Volume" MV reduziert sich somit bei auftretenden Leckage-Verlusten die Sprühzeit.

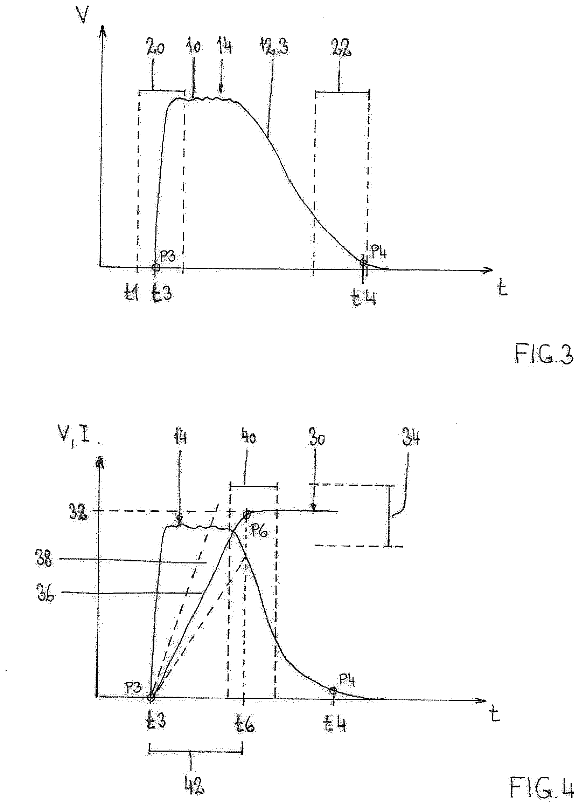

Darüber hinaus kann der Linienverlauf im Bereich des abfallenden Sprühstrahls 12 ausgewertet werden, da dieser sehr charakteristisch für die Qualität der Mikrodüse der zu prüfenden Baugruppe ist. Der Linienverlauf in Fig. 1 entspricht einer spezifikationsgerechten Baugruppe. In Fig. 2 und 3 sind dagegen die Linienverläufe von nicht-spezifikationsgerechten Baugruppen dargestellt. Sowohl bei der Messung in Fig. 2 als auch bei der Messung in Fig. 3 liegen die Zeitpunkte t3 und t4 innerhalb der vorgegebenen Referenzbereiche 20, 22. Allerdings fällt der Linienverlauf 12.2 in Fig. 2 deutlich steiler ab als bei der spezifikationsgerechten Baugruppe gemäß Fig. 1. Dies lässt auf eine zu große Öffnung der Mikrodüse schließen. In Fig. 3 fällt der Linienverlauf 12.3 dagegen deutlich langsamer ab als bei der spezifikationsgerechten Baugruppe gemäß Fig. 1. Dies lässt auf eine Verengung der Mikrodüse schließen, beispielsweise durch eine Verschmutzung der Mikrodüse.

Des Weiteren kann aus dem annähernd exponentiell abfallenden Linienverlauf im Bereich des abfallenden Sprühstrahls 12 eine Bestimmung der Halbwertszeit $t_h$ (50% des maximalen Verdunklungswerts) erfolgen (siehe Fig. 1). Aus dieser Halbwertszeit $t_h$ kann über die Formel

$$t_h = R_h \times C_h \times \ln(2)$$

der hydraulische Durchflusswiderstand $R_h$ der Düse bestimmt werden. Dabei kann angenommen werden, dass $C_h$ (hydraulische Kapazität oder Nachgiebigkeit des Pumpenkörpers) konstant ist. Daher kann bei einer hohen Halbwertszeit $t_h$ auf einen großen Durchflusswiderstand $R_h$ geschlossen werden. Da die Halbwertszeit $t_h$ bei einer konstant angenommenen Nachgiebigkeit des Pumpenkörpers unabhängig von den Antriebsgrößen (insbesondere der Feder und der Reibung innerhalb der Baugruppe) ist, ist diese sehr spezifisch für den Durchflusswiderstand $R_h$ der Düse.

Die gemessenen Verdunklungswerte können anschließend über die Dauer 24 des Sprühstrahls integriert werden. Das entsprechende Integral 30 kann dabei ebenfalls als Liniendiagramm ausgegeben werden, wie dies in Fig. 4 dargestellt ist. Im vorliegenden Beispielsfall sind dabei sowohl die eigentlichen Messwerte 14 als auch das Integral 30 in einem gemeinsamen Diagramm dargestellt. Im Gegensatz dazu könnten das Integral 30 auch in einem eigenständigen Diagramm ausgegeben werden.

Das ausgebrachte Volumen des Sprühstrahls 10, 12 ist proportional zum Endwert oder Maximum 32 des Integrals 30. Das Maximum 32 des Integrals 30 muss dabei in einem vorgegebenen Referenzbereich 34 liegen, damit es sich um eine spezifikationsgerechte Baugruppe handeln kann.

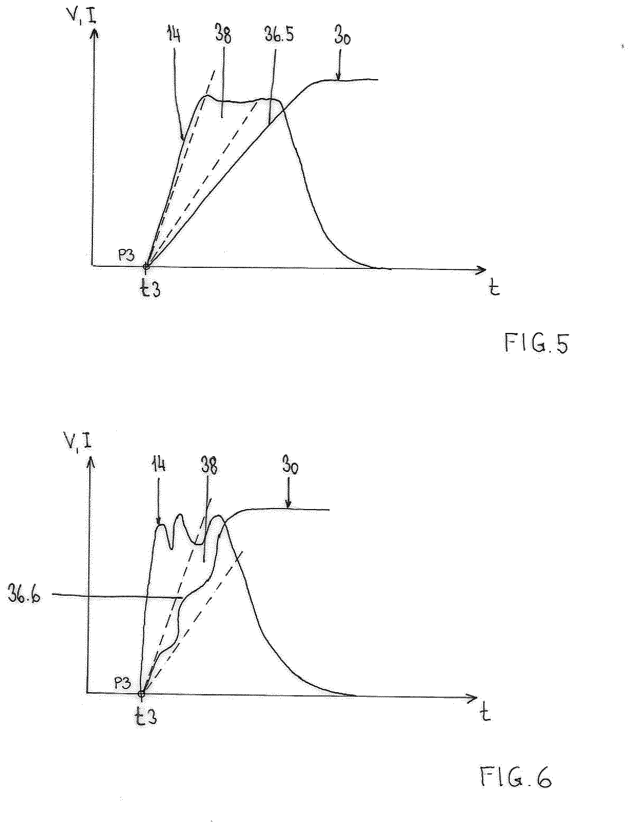

Darüber hinaus kann der ansteigende Linienverlauf 36 des Integrals 30 während des Sprühstrahls 10 ausgewertet werden. Der Linienverlauf in Fig. 4 entspricht einer spezifikationsgerechten Baugruppe. In Fig. 5 und 6 sind dagegen die Linienverläufe von nicht-spezifikationsgerechten Baugruppen dargestellt. So liegt der ansteigende Linienverlauf 36.5 in Fig. 5 außerhalb des vorgegebenen Referenzbereichs 38. In Fig. 6 liegt der Linienverlauf 36.6 zwar innerhalb des vorgegebenen Referenzbereichs 38, allerdings ist die Steigung des Linienverlaufs 36.6 nicht konstant. Vielmehr weist der Linienverlauf 36.6 mehrere Dellen auf. Dies lässt darauf schließen, dass der Kolben der zu prüfenden Baugruppe nicht stetig läuft. Die zu prüfende Baugruppe ist somit nicht spezifikationsgerecht und kann daher als "Schlecht" ausgegeben werden.

Darüber hinaus kann aus dem Integral 30 der Punkt P6 (Zeitpunkt t6) bestimmt werden. Hierbei handelt es sich um einen definierten Prozentbereich des Maximums 32 des Integrals 30, der als Ende des Sprühstrahls definiert werden kann. Dieser Zeitpunkt t6 muss ebenfalls in einem vorgegebenen Referenzbereich 40 liegen. In einem zweiten Schritt kann der zeitliche Abstand 42 zwischen dem Beginn des Sprühstrahls (Zeitpunkt t3) und dem Zeitpunkt t6 berechnet werden. Dieser zeitliche Abstand 42 muss in einem vorgegebenen Referenzbereich liegen, wenn es sich um eine spezifikationsgerechte Baugruppe handelt.

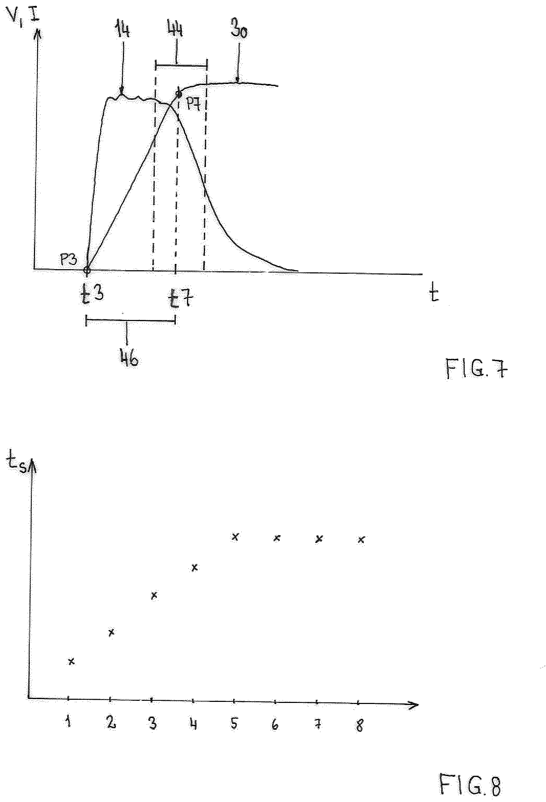

Alternativ oder zusätzlich zur Bestimmung des Zeitpunkts t6 kann auch der Übergangspunkt P7 des Integrals (Zeitpunkt t7) bestimmt werden (siehe Fig. 7). Der Übergangspunkt P7 des Integrals 30 muss ebenfalls in einem vorgegebenen Referenzbereich 44 liegen. Auch P7 kann zur Bestimmung der Sprühzeit herangezogen werden, indem der zeitliche Abstand 46 zwischen dem Beginn des Sprühstrahls (Zeitpunkt t3) und dem Punkt P8 berechnet wird. Auch hier gilt wieder, dass der zeitliche Abstand 46 in einem vorgegebenen Referenzbereich liegen muss, wenn es sich um eine spezifikationsgerechte Baugruppe handelt.

Wahlweise können die tatsächlich gemessenen Verdunklungswerte 14 und/oder die Auswerteergebnisse wie insbesondere das Integral 30 grafisch über ein XY-Diagramm an einem HMI ausgegeben werden. Die gespeicherten Verdunklungswerte und Auswerteergebnisse können darüber hinaus in einer tabellarischen Form in einem CSV-File ausgegeben werden.

Für eine Lebensdauer-Prüfung der zu prüfenden Baugruppe kann der komplette Prüfzyklus beliebig oft wiederholt werden. Alle Einzelmessungen, Auswerteergebnisse der einzelnen Prüfungen und das Gesamtergebnis können in Echtzeit erfasst, auf dem HMI angezeigt und/oder als CSV-File ausgegeben werden.

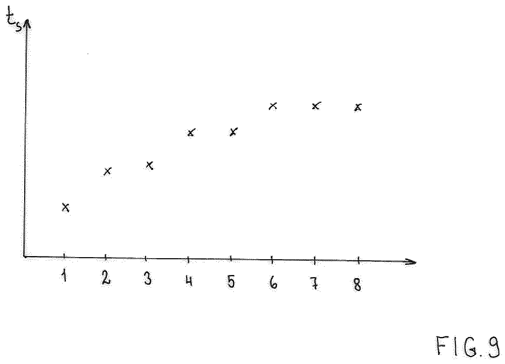

Auch die Qualität des Primens (Entlüften der Pumpe beim ersten Befüllen der zunächst trockenen Pumpenkammer) kann erfasst und bewertet werden. So kann beispielsweise ermittelt werden, mit wie vielen Auslösungen der Baugruppe die volle Sprühzeit erreicht wird (siehe Fig. 8 und 9). In Fig. 8 ist eine spezifikationsgerechte Baugruppe dargestellt. In diesem Fall steigt die Sprühzeit $t_s$ innerhalb der ersten fünf Primehübe annähernd konstant an. Anschließend bleibt die Sprühzeit $t_s$ annähernd konstant. Demgegenüber verläuft der Anstieg der Sprühzeit $t_s$ bei dem Diagramm gemäß Fig. 9 nicht konstant. Die Gleichförmigkeit des Anstiegs kann somit als Qualitätsmerkmal herangezogen werden, was bei einem Diagramm vergleichbar zu Fig. 9 zu einem Ausschluss der nicht-spezifikationsgerechten Baugruppe führen würde. Auf diese Weise kann beispielsweise ein schlecht arbeitendes Rückschlagventil aufgrund eines schlechten Ansprechens oder Dichtens entdeckt werden.

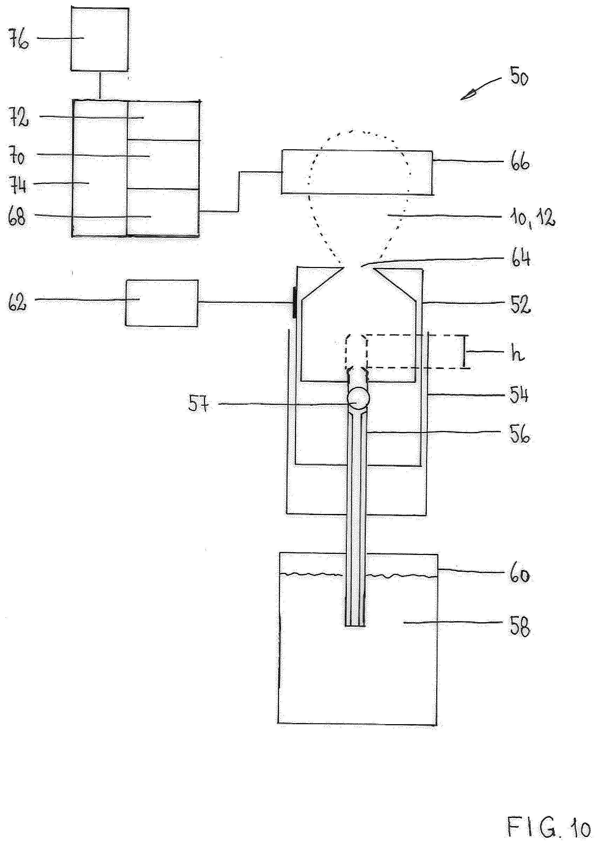

Fig. 10 zeigt eine schematische Darstellung der erfindungsgemäßen Vorrichtung 50 zum Prüfen des Sprühstrahls 10, 12 einer Baugruppe 52 für Sprühapplikatoren. Die Vorrichtung 50 besitzt eine Aufnahmeeinheit 54, in die die zu prüfende Baugruppe 52 eingesetzt werden kann. Im vorliegenden Beispielsfall kann die zu prüfende Baugruppe 52 mit ihrer Kapillare 56 und dem Rückschlagventil 57 an einem mit einem Prüf-Fluid 58 gefüllten Flüssigkeitsreservoir 60 angeschlossen werden. Bei dem Flüssigkeitsreservoir 60 kann es sich beispielsweise um eine Prüf-Kartusche handeln. Alternativ dazu kann das Flüssigkeitsreservoir 60 auch größer ausgebildet sein, so dass mehrere Aufnahmeeinheiten 54 an einem gemeinsamen Flüssigkeitsreservoir 60 angeschlossen werden können.

Die Vorrichtung 50 besitzt eine Betätigungseinheit 62, über die die zu prüfende Baugruppe 52 gespannt und betätigt werden kann, so dass ein Sprühstrahl 10, 12 aus der Düse 64 des Sprühapplikators freigesetzt wird. Der Sprühstrahl 10, 12 trifft dabei auf ein vor der Düse 64 positioniertes Lichtband 66, das durch den Sprühstrahl 10, 12 verdunkelt wird. Die Verdunklungswerte 14 werden von der Sensoreinheit 68 gemessen und im vorliegenden Beispielsfall auch gespeichert. Darüber hinaus ist eine Integriereinheit 70 vorhanden, mittels der die aufgezeichneten Messwerte 14 integriert werden können. Bei der Integriereinheit 70 kann es sich insbesondere um eine entsprechende Software handeln, die beispielsweise in einer Steuereinheit integriert sein kann. Im vorliegenden Beispielsfall werden sowohl die aufgezeichneten Messwerte 14 als auch das Integral 30 der aufgezeichneten Messwerte von einer Auswerteeinheit 72 ausgewertet. Die ermittelten Messwerte 14 sowie das Integral 30 der ermittelten Messwerte 14 wird an einer Anzeigeeinheit 74 angezeigt.

Darüber hinaus kann eine Steuereinheit 76 vorhanden sein. Diese Steuereinheit 76 kann gegebenenfalls auch einer übergeordneten Vorrichtung, insbesondere einer Montagevorrichtung für die zu prüfenden Baugruppen zugeordnet sein. Durch die Steuereinheit 76 können die ermittelten Gut/Schlecht-Bewertungen den einzelnen Baugruppe zugeordnet und deren Weiterverarbeitung entsprechend gesteuert werden.

**Patentansprüche**

1. Verfahren zum Prüfen des Sprühstrahls (10, 12) einer Baugruppe für Sprühapplikatoren mit folgenden Verfahrensschritten:

   - die zu prüfende Baugruppe wird in einer Aufnahmeeinheit einer Prüfvorrichtung positioniert und mit einem Flüssigkeitsreservoir verbunden,
   - die zu prüfende Baugruppe wird gespannt und saugt dabei ein bestimmtes Volumen des Prüf-Fluids aus dem Flüssigkeitsreservoir an,
   - die zu prüfende Baugruppe wird betätigt, so dass ein Sprühstrahl (10, 12) entsteht,
   - der Sprühstrahl (10, 12) verdunkelt ein Lichtband oder einen Lichtstrahl einer Lichtschranke,
   - die Verdunklung des Lichtbands beziehungsweise des Lichtstrahls wird mittels einer Sensoreinheit gemessen und die Messwerte (14) in Abhängigkeit von der Zeit registriert und ausgewertet,
   - aufgrund der ermittelten Messwerte (14) wird der Beginn (t3) des Sprühstrahls (10) und/oder das Ende (t4) des Sprühstrahls (12) und/oder die Dauer (24) des Sprühstrahls (10, 12) berechnet.

2. Verfahren nach Anspruch 1,

   - **dadurch gekennzeichnet, dass**
   - kontrolliert wird, ob der Beginn (t3) des Sprühstrahls (10) und/oder das Ende (t4) des Sprühstrahls (12) in einem vorgegebenen Referenzbereich (20, 22) liegt oder nicht.

3. Verfahren nach Anspruch 1 oder 2,

   - **dadurch gekennzeichnet, dass**
   - kontrolliert wird, ob die Dauer (24) des Sprühstrahls (10, 12) in einem vorgegebenen Referenzbereich liegt oder nicht.

4. Verfahren nach einem der vorstehenden Ansprüche,

   - **dadurch gekennzeichnet, dass**

- die Verdunklungswerte (14) in Form eines Diagramms, insbesondere in Form eines Liniendiagramms, und/oder tabellarisch ausgegeben werden.

5. Verfahren nach Anspruch 4,

- **dadurch gekennzeichnet, dass**
- kontrolliert wird, ob der abfallende Linienverlauf zum Ende des Sprühstrahls (12) hin in einem vorgegebenen Referenzbereich liegt oder nicht.

6. Verfahren nach einem der vorstehenden Ansprüche,

- **dadurch gekennzeichnet, dass**
- die ermittelten Messwerte (14) zumindest im Bereich zwischen dem Beginn (t3) des Sprühstrahls (10) und dem Ende (t4) des Sprühstrahls (12) integriert werden,
- das Integral (30) der Messwerte (14) anschließend ausgewertet wird.

7. Verfahren nach Anspruch 6,

- **dadurch gekennzeichnet, dass**
- das Integral (30) der Messwerte (14) in Form eines Liniendiagramms und/oder tabellarisch ausgegeben wird.

8. Verfahren nach Anspruch 7,

- **dadurch gekennzeichnet, dass**
- kontrolliert wird, ob der Anstieg (36, 36.5, 36.6) des Integrals (30) in einem vorgegebenen Referenzbereich (38) liegt oder nicht.

9. Verfahren nach einem der Ansprüche 6 bis 8,

- **dadurch gekennzeichnet, dass**
- kontrolliert wird, ob der Anstieg (36, 36.5, 36.6) des Integrals (30) etwa linear verläuft oder nicht.

10. Verfahren nach einem der Ansprüche 6 bis 9,

- **dadurch gekennzeichnet, dass**
- kontrolliert wird, ob das Maximum (32) des Integrals (30) in einem vorgegebenen Referenzbereich (34) liegt oder nicht.

11. Verfahren nach einem der Ansprüche 6 bis 10,

- **dadurch gekennzeichnet, dass**
- der Übergangspunkt (P7) des Integrals (30) bestimmt wird.

12. Verfahren nach einem der Ansprüche 6 bis 11,

- **dadurch gekennzeichnet, dass**
- das Überschreiten einer definierten Prozentzahl des Maximus (32) des Integrals (30) als Ende (P6) des Sprühstrahls (10) bestimmt wird.

13. Verfahren nach Anspruch 11 oder 12,

- kontrolliert wird, ob der Übergangspunkt (P7) beziehungsweise das Ende (P6) des Sprühstrahls (10) in einem vorgegebenen Referenzbereich (44, 40) liegt oder nicht.

14. Verfahren nach einem der Ansprüche 11 bis 13,

- der zeitliche Abstand (46, 42) zwischen dem Übergangspunkt (P7) des Integrals (30) beziehungsweise dem Ende (P6) des Sprühstrahls (10) und dem Beginn des Sprühstrahls (P3) berechnet wird,
- kontrolliert wird, ob der zeitliche Abstand (46, 42) in einem vorgegebenen Referenzbereich liegt oder nicht.

15. Verfahren nach Anspruch 1,

- **dadurch gekennzeichnet, dass**
- die Verfahrensschritte mehrmals hintereinander durchgeführt werden,
- für jede Messung die Dauer (24) des Sprühstrahls (10, 12) berechnet wird,
- die ermittelten Sprühstrahl-Dauern (24) der einzelnen Messungen in der Reihenfolge der Messungen ausgewertet werden.

16. Verfahren nach Anspruch 15,

- **dadurch gekennzeichnet, dass**
- die ermittelten Sprühstrahl-Dauern (24) in Form eines Diagramms, insbesondere in Form einen Liniendiagramms, und/oder tabellarisch ausgegeben werden.

17. Vorrichtung (50) zum Prüfen des Sprühstrahls (10, 12) einer Baugruppe (52) für Sprühapplikatoren,

- mit zumindest einer Aufnahmeeinheit (54) für die zu prüfende Baugruppe (52),
- mit zumindest einem Flüssigkeitsreservoir (60) für ein Prüf-Fluid (58),
- mit einer Betätigungseinheit für die zu prüfende Baugruppe (52),
- mit einer Lichtschranke mit einem Lichtband (66) oder einem Lichtstrahl, die so vor der zu prüfenden Baugruppe (52) positioniert ist, dass der von der zu prüfenden Baugruppe (52) erzeugte Sprühstrahl (10, 12) auf das Lichtband (66) beziehungsweise den Lichtstrahl trifft,
- mit einer Sensoreinheit (68), durch die die Verdunklung des Lichtbandes (66) beziehungsweise des Lichtstrahls messbar ist,
- mit einer Integriereinheit (70), zum Integrieren der aufgezeichneten Messwerte (14),
- mit zumindest einer Auswerteeinheit (72) zum Auswerten der aufgezeichneten Messwerte (14) und dem Integral (30) der aufgezeichneten Messwerte (14).

18. Vorrichtung nach Anspruch 17,

- **dadurch gekennzeichnet, dass**
- mit einer Anzeigeeinheit (74) zum Ausgeben der aufgezeichneten Messwerte (14) und/oder zum Ausgeben des Integrals (30) der aufgezeichneten Messwerte (14), insbesondere in Form von Liniendiagrammen und/oder in Tabellenform, vorhanden ist.

19. Vorrichtung nach Anspruch 17 oder 18,

- **dadurch gekennzeichnet, dass**
- die Lichtschranke einen Laserstrahl oder einen gefächerten Laserstrahl aufweist.

FIG.1

FIG.2

FIG.3

FIG.4

FIG.5

FIG.6

FIG.7

FIG.8

FIG.9

FIG. 10

Europäisches Patentamt
European Patent Office
Office européen des brevets

# EUROPÄISCHER RECHERCHENBERICHT

**Nummer der Anmeldung**

EP 24 18 4279

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 6 785 400 B1 (FARINA DINO J [US]) 31. August 2004 (2004-08-31) * Abbildung 1 * * Spalte 1, Zeile 26 - Spalte 2, Zeile 6 * * Spalte 2, Zeile 53 - Zeile 67 * * Spalte 4, Zeile 15 - Zeile 22 * * Spalte 5, Zeile 29 - Zeile 32 * * Spalte 5, Zeile 42 - Zeile 47 * * Spalte 6, Zeile 46 - Zeile 49 * ----- | 1-19 | INV. G01F25/00 ADD. A61M15/08 B05B11/00 |
| A | WO 2017/060328 A1 (BOEHRINGER INGELHEIM MICROPARTS GMBH [DE]) 13. April 2017 (2017-04-13) * Seite 2, Zeile 25 - Zeile 32 * * Seite 5, Zeile 4 - Zeile 10 * * Seite 5, Zeile 15 - Zeile 16 * * Seite 16, Zeile 35 - Seite 17, Zeile 8 * * Seite 29, Zeile 18 - Zeile 21 * * Seite 30, Zeile 17 - Zeile 35 * ----- | 1-19 | |
| A | EP 2 381 237 B1 (BOEHRINGER INGELHEIM MICROPARTS GMBH [DE]) 19. Juni 2019 (2019-06-19) * Absätze [0001], [0006], [0014], [0017], [0020], [0028] * ----- | 1-19 | RECHERCHIERTE SACHGEBIETE (IPC) A61M B05B G01F |
| A | WO 01/13322 A1 (IMAGE THERM ENGINEERING [US]) 22. Februar 2001 (2001-02-22) * Seite 6, Zeile 29 - Seite 7, Zeile 12 * ----- | 1-19 | |
| A | US 2023/123194 A1 (BOISVILLIERS JULIEN [FR]) 20. April 2023 (2023-04-20) * Absätze [0060], [0061] * ----- -/-- | 1-19 | |

Der vorliegende Recherchenbericht wurde für alle Patentansprüche erstellt

| Recherchenort | Abschlußdatum der Recherche | Prüfer |
|---|---|---|
| Den Haag | 27. November 2024 | Nierhaus, Thomas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

...............................................................................
& : Mitglied der gleichen Patentfamilie, übereinstimmendes Dokument

EPO FORM 1503 03.82 (P04C03)

Europäisches
Patentamt
European
Patent Office
Office européen
des brevets

# EUROPÄISCHER RECHERCHENBERICHT

**Nummer der Anmeldung**

EP 24 18 4279

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | LINNE MARK: "Imaging in the optically dense regions of a spray: A review of developing techniques", PROGRESS IN ENERGY AND COMBUSTION SCIENCE., Bd. 39, Nr. 5, 29. Juni 2013 (2013-06-29), Seiten 403-440, XP093227537, NL ISSN: 0360-1285, DOI: 10.1016/j.pecs.2013.06.001 * das ganze Dokument * ----- | 1-19 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| Den Haag | 27. November 2024 | Nierhaus, Thomas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**   EP 24 18 4279

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-11-2024

| Im Recherchenbericht angeführtes Patentdokument | Datum der Veröffentlichung | Mitglied(er) der Patentfamilie | Datum der Veröffentlichung |
|---|---|---|---|
| US 6785400 B1 | 31-08-2004 | US 6785400 B1 | 31-08-2004 |
| | | US 2004258278 A1 | 23-12-2004 |
| | | US 2009136086 A1 | 28-05-2009 |
| WO 2017060328 A1 | 13-04-2017 | CA 2997649 A1 | 13-04-2017 |
| | | CN 108449935 A | 24-08-2018 |
| | | EP 3359945 A1 | 15-08-2018 |
| | | HK 1252032 A1 | 10-05-2019 |
| | | IL 258063 A | 31-05-2018 |
| | | JP 6991129 B2 | 03-02-2022 |
| | | JP 2018536841 A | 13-12-2018 |
| | | US 2019113418 A1 | 18-04-2019 |
| | | WO 2017060328 A1 | 13-04-2017 |
| EP 2381237 B1 | 19-06-2019 | EP 2381237 A1 | 26-10-2011 |
| | | EP 3537132 A1 | 11-09-2019 |
| | | PT 2381237 T | 08-10-2019 |
| WO 0113322 A1 | 22-02-2001 | AU 6646500 A | 13-03-2001 |
| | | CA 2382252 A1 | 22-02-2001 |
| | | EP 1210683 A1 | 05-06-2002 |
| | | JP 4394322 B2 | 06-01-2010 |
| | | JP 2003525658 A | 02-09-2003 |
| | | TW 487884 B | 21-05-2002 |
| | | US 6665421 B1 | 16-12-2003 |
| | | US 2004131243 A1 | 08-07-2004 |
| | | US 2006034504 A1 | 16-02-2006 |
| | | WO 0113322 A1 | 22-02-2001 |
| US 2023123194 A1 | 20-04-2023 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9114468 A1
- WO 9712687 A1
- WO 2009047173 A2
- WO 2017060328 A1
- US 2019223418 A1
- DE 2020210028 U1
- EP 4109066 A1